(19) Europäisches Patentamt
European Patent Office
Office européen des brevets

(11) Publication number: **0 382 814 B1**

(12) # EUROPEAN PATENT SPECIFICATION

(45) Date of publication of patent specification: **16.02.94**  (51) Int. Cl.⁵: **A23C 19/11**, A61K 37/02

(21) Application number: **89907595.6**

(22) Date of filing: **16.06.89**

(86) International application number:
**PCT/US89/02625**

(87) International publication number:
**WO 89/12399 (28.12.89 89/30)**

Divisional application 93200152.2 filed on
16/06/89.

The file contains technical information submitted
after the application was filed and not included in
this specification

(54) **NISIN COMPOSITIONS FOR USE AS ENHANCED, BROAD RANGE BACTERICIDES.**

(30) Priority: **22.06.88 US 209861**
**01.03.89 US 317626**

(43) Date of publication of application:
**22.08.90 Bulletin 90/34**

(45) Publication of the grant of the patent:
**16.02.94 Bulletin 94/07**

(84) Designated Contracting States:
**AT BE CH DE FR GB IT LI LU NL SE**

(56) References cited:
**EP-A- 0 384 319**
**GB-A- 738 655**

(73) Proprietor: **APPLIED MICROBIOLOGY, INC.**
**170 53rd Street**
**Brooklyn, N.Y. 11232(US)**

(72) Inventor: **BLACKBURN, Peter**
**426 W. 44th Street**
**New York, NY 10036(US)**
Inventor: **POLAK, June**
**57 Montague Street**
**Brooklyn, NY 11201(US)**
Inventor: **GUSIK, Sara-Ann**
**317 First Avenue**
**New York, NY 10003(US)**
Inventor: **RUBINO, Stephen, D.**
**111 Henry Avenue**
**Harrison, NY 10528(US)**

EP 0 382 814 B1

**Chemical Abstracts, vol. 86, no. 1, 3 January 1977 (Colombus, Ohio, US), A.I. Pedenko et al.:"Effect of the antibiotic nisin on pathogenic staphylococci and streptococci"**

**Römpps Chemie-Lexikon, p. 1126, 644 of the 8th Edition**

**Kirk-Othmer Encyclopedia of Chem. Technology, third Edition, vol. 6, p. 150, 154**

(74) Representative: **Lucas, Brian Ronald et al**
**Lucas & Co.**
**135 Westhall Road**
**Warlingham Surrey CR6 9HJ (GB)**

## Description

### Background of the Invention

Nisin is a Lanthionine-containing polypeptide with antimicrobial properties which is produced in nature by various strains of the bacterium Streptococcus lactis. It is a known food preservative which inhibits the outgrowth of spores of certain species of Gram positive Bacilli.

Although sometimes mistakenly and imprecisely referred to as an antibiotic, nisin is more correctly classified as a bacteriocin, i.e. a proteinaceous substance produced by bacteria and which has antibacterial activity only towards species closely related to the species of its origin. Nisin is a naturally-occurring preservative found in low concentration in milk and cheese, and is believed to be completely non-toxic and non-allergenic to humans.

Nisin has recently been recognized as safe by the FDA as a direct food ingredient in pasteurized cheese spread, pasteurized processed cheese spread, and pasteurized or pasteurized processed cheese spread with fruits, vegetables, or meats. Furthermore, since it is a polypeptide, any nisin residues remaining in foods are quickly digested.

A summary of nisin's properties appears in Hurst, A., Advances in Applied Microbiology 27:85-123 (1981). This publication describes what is generally known about nisin. Nisin, produced by Streptococcus lactis, is available commercially as an impure preparation, Nisaplin™, from Aplin & Barrett Ltd., Dorset, England and can be obtained by isolating naturally-occurring nisin from cultures of Streptococcus lactis and then concentrating the nisin according to known methods. There are also reported methods for producing nisin using altered strains of Streptococcus. See Gonzalez et al., U.S. Pat. No. 4,716,115, issued December 29, 1987. It should also be possible to produce nisin by recombinant DNA technology.

Nisin has been applied effectively as a preservative in dairy products, such as processed cheese, cream and milk. The use of nisin in processed cheese products has been the subject of recent patents. See U.S. Pat. Nos. 4,584,199 and 4,597,972. The use of nisin to inhibit the growth of certain Gram positive bacteria has been well documented. However, its complete success and acceptance as a food preservative has heretofore been hampered by the belief that nisin was ineffective against Gram negative and many Gram positive bacteria. Gram negative bacteria are almost always present in conjunction with Gram positive bacteria and are a major source of food spoilage and contamination. See Taylor, U.S. Pat. No. 5,584,199, issued April 22, 1986 and Taylor, U.S. Pat. No. 4,597,972, issued July 1, 1986; Tsai and Sandine, "Conjugal Transfer of Nisin Plasmid Genes from Streptococus Lactis 7962 to Leuconostoc Dextranicum 181, Applied and Environmental Microbiology, Feb. 1987, p. 352; "A Natural Preservative," Food Engineering Int'l,, May 1987, pp. 37-38; "Focus on Nisin," Food Manufacture, March 1987, p. 63.

### Summary of the Invention

It has now been found that contrary to prior teaching, compositions comprising nisin, in combination with a chelating agent have enhanced, broad range bactericidal activity against Gram negative bacteria as well as enhanced activity against a broader range of Gram positive bacteria than nisin alone. The enhanced bactericidal activity against Gram positive bacteria occurs in a pH range broader than previously taught. The invention provides compositions for enhancing the performance of nisin or another lanthionine-containing bacteriocin, comprising said bacteriocin in combination with a chelating agent. The invention further provides the compositions dissolved or suspended in a suitable carrier to yield enhanced broad range bactericides. Various uses of the combination of a lanthionine-containing bacteriocin and chelating agent, including a method of treatement of food, all as described hereinafter, are also within the invention.

### Additional reference to prior art

British Patent 738,655 is directed to a dry preparation containing nisin for manufacturing cheese. In one embodiment, a concentrated nisin-containing whey is blended with cheese. When a very old, hard cheese is added, the addition of a calcium sequestering agent such as an alkali metal polyphosphate is recommended. An emulsifier such as citrate can be aded to make processed cheese. The resultant cheese blend is not considered to be a composition within the scope of the present invention, since calcium salts, present in great excess in the blend, will take up all the effective chelating capacity of the sequestering agent or citrate emulsifier, leaving it with no effective residual capacity to act as a chelating agent in combination with nisin. Further, this specification does not refer to any enhancement of the bactericidal activity of nisin.

Detailed Description of the Invention

Specifically, it has been found that a solution of about 0.1 $\mu$g/ml to 300 $\mu$g/ml of nisin in the presence of about 0.1 mM to 20 mM of a chelating agent, for example EDTA, virtually eliminates the growth of Gram negative bacteria such as Salmonella typhimurium, Escherichia coli, Pseudomonas aeruginosa, Bacterioides gingivalis, Actinobacillus actinomycetescomitans, and Klebsiella pneumoniae and is more active towards Gram positive bacteria such as Staphylococcus aureus, Streptococcus mutans, Listeria monocytogenes Streptococcus agalactiae and Coryneform bacteria than nisin alone. Although the enhancement of nisin activity by chelator was concentration dependent, contrary to expectations, concentrations of EDTA in excess of 20mM were inhibitory to the bactericidal activity of nisin. However, in the presence of a proteinaceous carrier, and polyvalent polymers such as serum albumin, collagen, gelatin, casein and keratin, the inhibition of nisin by concentrations of EDTA above 20mM was significantly reduced, thereby extending the useful range of EDTA enhancement of nisin.

It has also been found that a solution of about 0.1 $\mu$g/ml to 300 $\mu$g/ml nisin and about 0.1 mM to 20 mM of a chelating agent will further enhance the effectiveness of nisin against Gram negative and Gram positive bacteria in the presence of about 0.01% to 1.0% of surfactant.

In the present invention, suitable chelating agents include, but are not limited to, EDTA, CaEDTA, CaNa$_2$EDTA, and other alkylenediamine tetraacetates, EGTA and citrate. Surfactants, valuable as cleansing agents, suitable for combination with nisin, with EDTA, include, but are not limited to, the nonionic surfactants Tweens, Tritons, and glycerides, ionic surfactants such as fatty acids, quaternary compounds, anionic surfactants and amphoteric surfactants such as cocamidopropyl betaine and emulsifiers.

Since Gram positive and Gram negative bacteria are almost always found together in foods, the effectiveness of the nisin compositions towards Gram negative bacteria such as Salmonella typhimurium, Escherichia coli, Klebsiella pneumoniae, Pseudomonas aeruginosa, Bacterioides gingivalis, Actinobacillus actinomycetescomitans, and other Gram negative pathogens and Gram positive bacteria will be of great use. The bactericides are particularly suited for the control and prevention of contamination of raw ingredients, processed foods and beverages by bacterial pathogens and other microbial spoilage organisms. Potential food related uses include treatment of meats, especially poultry, eggs, cheese and fish and treatment of food packaging and handling equipment. Further uses include as food preservative, such as in processed cheese, cream, milk, dairy products and in cleaning poultry, fish, meats, vegetables, and dairy and food processing equipment. The use of the nisin compositions should not be limited to food related uses and the nisin compositions should be useful in any situation in which there is a need or desire to eliminate Gram negative and Gram positive bacteria.

The compositions can be dissolved in a suitable carrier for example an aqueous solvent or buffer or suspended in any suitable liquid, colloidal or polymeric matrix to create bactericides. The compositions or bactericides can be incorporated into ointments or coatings for medicinal uses such as the treatment of infections, wound dressings or surgical implants and as a broad spectrum disinfectant for skin or oral rinses, disinfectant scrubs, wipes or lotions. The bactericides can be used for cleaning medical instruments, in pre-operative surgical scrubs and the like. The bactericides are particularly useful in circumstances where environmental disinfection is desired but where chemical germicidals are precluded because of the risks of corrosive or otherwise toxic residues.

Unlike the activity of most broad spectrum germicidals which is compromised by the presence of complex organic matter, the compositions of the present invention are effective as bactericides in the presence of organic matter, such as milk or serum.

Nisin was known to optimally inhibit the growth of a few closely related Gram positive bacteria, particularly certain Gram positive spore forming bacilli at pH 5.0. The bactericidal activity of nisin in solution with a chelating agent was surprisingly rapid and greatly enhanced towards a broad range of Gram positive bacteria at pH values greater than pH 5.0, and, moreover, was activated towards Gram negative bacteria at both acidic and basic pH, preferably in the range pH 5.0 to 8.0. This unexpectedly rapid and broad-ranged bactericidal activity of chelator-activated nisin makes it suitable for use as, among other things, a disinfectant.

Nisin belongs to the class of peptide bacteriocins containing lanthionine. Also included among that class are subtilin, epidermin, cinnamycin, duramycin, ancovenin and Pep 5. These bacteriocin peptides are each produced by different microorganisms. However, subtilin obtained from certain cultures of Bacillus subtilis, and epidermin obtained from certain cultures of Staphylococcus epidermidis, have been found to have molecular structures very similar to that of nisin (see Hurst, pp. 85-86, and Schnell et al., Nature, 333:276-278). It is therefore believed that because of the molecular similarities, other lanthionine-containing peptide bacteriocins will be equally as effective as nisin in combination with chelating agents in eliminating Gram negative and Gram positive bacterial contaminations.

The effectiveness of the nisin, and by extension other lanthionine-containing peptide bacteriocin, compositions as bactericides against Gram negative bacteria is surprising, since the prior art generally teaches away from this activity of nisin. The enhanced activity of nisin against Gram positive bacteria in the presence of EDTA at a pH greater than 5.0 is unexpected since it was previously believed that nisin activity is optimal at pH 5.0. Furthermore, the discovery of such effectiveness of the nisin and lanthionine-containing peptide bacteriocin compositions as bactericides fulfills a long-felt need in the science of food preservation, which has suffered from the absence of an acceptable, natural, non-toxic agent effective against a broad range of bacteria.

In order to demonstrate the superior and unexpected rapid activity of the composition containing nisin, EDTA and/or optionally various surfactants, against both Gram negative and Gram positive bacteria, a number of experiments were conducted with the bactericides. These experiments are meant as illustration and are not intended to limit this invention. It is to be expected that other, lanthionine-containing peptide bacteriocins would be effective substitutes for nisin and that chelating agents other than EDTA will be effective substitutes for EDTA.

All tests in the following examples were performed at 37°C. The efficacy of the enhanced broad range bactericides was determined by assaying bactericidal activity as measured by the percent bacterial survival after treatment with the bactericide. Generally, after incubation of a $10^7$ cell per ml suspension of target species with the novel bactericide for specified lengths of time, bacteria were collected by centrifugation for 2 minutes. The bacterial pellet was washed free of the bactericide with a rescue buffer, termed herein Phage buffer (50mM Tris-HCl buffer pH 7.8, 1mM $MgSO_4$, 4mM $CaCl_2$, 0.1 M Nacl, and 0.1% gelatin), resuspended and serially diluted into Phage buffer, and 100ml of the suspended bacteria were spread on nutrient agar plates. Surviving bacteria were determined by scoring colony forming units (CFU) after incubation for 24-48 hours at 37°C. An effective bactericide according to this invention is one which allows less than 0.1% of the initial viable count of the bacteria to survive.

Example 1

Activity of Nisin and a Chelating Agent Against Gram Negative Bacteria (S. typhimurium)

As shown in Table 1, two tests were conducted in 20mM Tris, pH 8.0 at 37°C to show the effect of the bactericide containing nisin and the chelating agent EDTA alone. Test #1, a control, was conducted without EDTA and shows the effect of nisin alone toward the Gram negative bacterium S. typhimurium. The increased concentrations of nisin do exhibit some activity, but even the activity of the higher concentrations in the absence of EDTA, 1.6% survival at 100 µg/ml nisin, is wholly inadequate for a food preservative. The level of bactericidal activity obtained from nisin and EDTA is significant.

TABLE 1

| Test # | Initial Viable Bacteria Count | EDTA (mM) | Nisin (µg/ml) | | | | | |
|---|---|---|---|---|---|---|---|---|
| | | | 0 | 10 | 30 | 50 | 100 | 300 |
| | | | Percentage S. typhimurium Survival at 3 hours | | | | | |
| 1 | $3.0 \times 10^6$ | 0 | 100 | 51.3 | - | 7.0 | 1.6 | - |
| 2 | $5.7 \times 10^6$ | 20 | 2.5 | - | $10^{-3}$ | - | $<10^{-4}$ | $<10^{-4}$ |

Test #2 (Table 1), conducted using nisin plus 20mM EDTA, demonstrates the surprising activity of the nisin composition in eliminating the target Gram negative bacteria.

Table 1 shows that in test #2 at a concentration of 20mM EDTA and 30 µg/ml of nisin, the bactericide has a marked bactericidal activity towards S. typhimurium, while at nisin concentrations of 100 µg/ml and greater, the nisin and EDTA bactericide virtually eliminates the bacteria (percentage survival less than $10^{-4}$ which indicates no surviving bacteria in the assay). Thus, the combination of EDTA and nisin demonstrates a synergistic activity of greater than 1000 times that of nisin alone.

5

Example 2

Activity of Nisin, a Chelating Agent and a Surfactant Against Gram Negative Bacteria (S. typhimurium)

Four tests (Table 2) were conducted to determine the effect on S. typhimurium of the bactericide containing nisin and both EDTA and the surfactant Triton X-100 in 20mM Tris, pH 8.0 at 37°. The control (Test #1) is identical to the control of Example 1 (Table 1).

TABLE 2

| Test # | Initial Viable Bacteria Count | EDTA (mM) | Triton X-100 (%) | Nisin (µg/ml) Percentage S. typhimurium Survival at 3 hours | | | | | |
|---|---|---|---|---|---|---|---|---|---|
| | | | | 0 | 10 | 30 | 50 | 100 | 300 |
| 1 | $3.0 \times 10^6$ | 0 | 0 | 100 | 51.3 | – | 7.0 | 1.6 | – |
| 2 | $3.0 \times 10^6$ | 0 | 1.0 | 37.4 | 93.0 | – | 64.0 | 47.0 | – |
| 3 | $5.7 \times 10^6$ | 20 | 0.1 | 0.03 | – | $<10^{-3}$ | – | – | – |
| 4 | $5.7 \times 10^6$ | 20 | 1.0 | $<10^{-4}$ | – | $<10^{-4}$ | – | $<10^{-4}$ | $<10^{-4}$ |

Test #2 (Table 2) was conducted using nisin and 1.0% Triton X-100, but without EDTA. The presence of the detergent alone inhibits the activity of the nisin towards the Gram negative bacteria and nisin was ineffective. However, in tests #3 and #4 (Table 2), which represent the invention, the presence of 20mM EDTA in combination with Triton X-100 is a bactericide which markedly increases the bactericidal activity of nisin towards S. typhimurium. Indeed the combination of Triton X-100 with EDTA but without nisin was

7

effective, although to a lesser degree than in the presence of nisin. While in both tests #3 and #4 (Table 2) the nisin combinations were very effective, the concentration of 1.0% Triton X-100 (test #4, Table 2) was most effective.

The presence of the non-ionic surfactant, Triton X-100, in combination with EDTA, enhances the activity of nisin toward Gram negative bacteria even more than the bactericide containing nisin and EDTA alone (Example 1).

Example 3

Activity of Nisin, a Chelating Agent and a Surfactant Against Gram Negative Bacteria (S. typhimurium)

Table 3 shows the enhanced activity toward S. typhimurium of the bactericide containing nisin, 20mM of the chelating agent EDTA and the non-ionic surfactant Tween 20 in 20 mM Tris, pH 8.0 at 37°C. As with Triton X-100 (Example 2) the combination of nisin and EDTA with (1%) of Tween 20 is most effective.

TABLE 3

| Test # | Initial Viable Bacteria Count | EDTA (mM) | Tween20 (%) | Nisin ($\mu$g/ml) | | | | | |
|---|---|---|---|---|---|---|---|---|---|
| | | | | 0 | 10 | 30 | 50 | 100 | 300 |
| | | | | Percentage S. typhimurium Survival at 3 hours | | | | | |
| 1 | $3.0 \times 10^6$ | 0 | 0 | 100 | 51.3 | – | 7.0 | 1.6 | – |
| 2 | $5.7 \times 10^6$ | 20 | 0 | 2.5 | – | $<10^{-3}$ | – | $<10^{-4}$ | $<10^{-4}$ |
| 3 | $4.3 \times 10^6$ | 20 | 1.0 | $<10^{-2}$ | – | $<10^{-4}$ | – | $<10^{-4}$ | $<10^{-4}$ |

Example 4

Activity of Nisin, a Chelating Agent and a Surfactant Against Gram Negative Bacteria (Escherichia coli)

The effect of the bactericide containing nisin and EDTA towards the Gram negative bacteria E. coli was demonstrated, as shown in Table 4.

TABLE 4

| Test # | Initial Viable Bacteria Count | EDTA (mM) | Triton X-100 (%) | Nisin ($\mu$g/ml) | | | |
|---|---|---|---|---|---|---|---|
| | | | | 0 | 30 | 100 | 300 |
| | | | | Percentage E. coli Survival at 2 hours | | | |
| 1 | $1.0 \times 10^7$ | 0 | 0 | 100 | 27 | 25 | 8.5 |
| 2 | $1.0 \times 10^7$ | 20 | 0 | 14.5 | 0.86 | 0.01 | 0.001 |
| 3 | $1.0 \times 10^7$ | 0 | 1.0 | 100 | - | 30 | - |
| 4 | $1.0 \times 10^7$ | 20 | 1.0 | 1.2 | 0.8 | 0.05 | $<10^{-4}$ |

The tests, with and without EDTA, were performed in 20mM Tris buffer solution, pH 8.0 at 37°C, with an initial viable count of $1 \times 10^7$ E. coli cells/ml. The effects of the bactericide were measured as a function of percentage bacteria survival after 2 hours.

In test #1, (control, Table 4) without EDTA, nisin exhibited little meaningful activity toward the elimination of E. coli. In test #2 (Table 4), however, where 20mM EDTA was present, the bactericidal composition exhibited substantial activity towards the E. coli bacteria. The activity increased in effectiveness as the concentration of nisin was increased. The combination of nisin with EDTA as a bactericide demonstrates a 1000 fold synergistic increase in effectiveness towards E. coli. In tests #3 and #4 (Table 4), it can be seen that Triton X-100 has no significant bactericidal activity towards E. coli. In fact, Triton X-100 appears to inhibit nisin activity towards Gram negative bacteria as was found with S. typhimurium (Table 2). However, the overall enhancement of nisin by EDTA substantially reverses the inhibitory effects of Triton X-100 as seen in Tables 2 and 4.

It thus appears that the bactericide containing nisin and a chelating agent, such as EDTA, is an effective food preservative towards various types of Gram negative bacteria even in the presence of surfactants.

Example 5

Activity of Nisin and a Chelating Agent Against Gram Negative Bacteria (Klebsiella pneumoniae)

The effect of the bactericide containing nisin and EDTA alone towards the Gram negative bacteria K. pneumoniae was demonstrated, as shown in Table 5.

TABLE 5

| Test | Initial Viable Bacteria Count | EDTA (mM) | Triton X-100 (%) | Nisin $\mu$g/ml | | | |
|------|------|------|------|------|------|------|------|
| | | | | 0 | 30 | 100 | 300 |
| | | | | % Survival at 2 hours | | | |
| 1 | $10^7$ | 0 | 0 | 100 | - | 50 | 38 |
| 2 | $10^7$ | 20 | 0 | 22 | 0.5 | 1.1 | 0.085 |

The two tests, one with and one without EDTA (control), were performed in 20 mM Tris buffer, pH 8.0 at 37°C with an initial viable count of $10^7$ cells/ml of K. pneumoniae. The effect was measured as a function of percentage bacterial survival after 2 hours.

In test #1, (control, Table 5) without EDTA, nisin exhibited little meaningful bactericidal activity toward K. pneumoniae. In test #2 (Table 5), however, where 20mM EDTA was present, the bactericide exhibited substantial activity towards K. pneumoniae. The activity increased in effectiveness as the concentration of nisin was increased.

Example 6

Nisin Activity Against Gram Negative Bacteria (Salmonella typhimurium) is Dependent on Chelator Concentration

The data in Table 6 demonstrate that the enhanced activation of nisin towards Gram negative bacteria (S. typhimurium) is dependent on the concentration of EDTA in either 50 mM sodium acetate, pH 5.0, or 20 mM Tris, pH 8.0 at 37°C.

TABLE 6

| Test # | pH | Initial Viable Bacterial Count | Nisin μg/ml | EDTA(mM) % Survival at 2 hours | | | | | |
|---|---|---|---|---|---|---|---|---|---|
| | | | | 0 | 0.2 | 2.0 | 10 | 50 | 100 |
| 1 | 5.0 | $3 \times 10^6$ | 0 | 100 | – | 38.7 | 15.2 | 3.5 | – |
| 2 | 5.0 | $3 \times 10^6$ | 100 | 0.6 | $10^{-4}$ | $10^{-4}$ | 0.004 | 0.02 | – |
| 3 | 8.0 | $5 \times 10^6$ | 0 | 100 | – | 8.7 | 14 | 11.4 | 45 |
| 4 | 8.0 | $5 \times 10^6$ | 100 | 4 | $10^{-4}$ | $10^{-4}$ | $10^{-4}$ | 0.6 | 30 |

In tests #1 and #3, (controls, Table 6) using EDTA concentrations up to 100 mM without nisin exhibited little meaningful activity towards S. typhimurium at either pH 5.0 (#1) or pH 8.0 (#3). In tests #2 and #4 (Table 6), however, where 100 μg/ml nisin was present in combination with EDTA, the bactericides exhibited substantial activity towards S. typhimurium. The activity of the bactericides was similar at both acidic pH (5.0) and basic pH (8.0), despite the fact that the activity of nisin alone towards Gram positive

10

bacteria is optimal at pH 5.0.

The enhancement of nisin by EDTA was concentration dependent, being optimal in the range 0.2 mM to 10 mM at pH values 5.0 and 8.0. Surprisingly, at concentrations greater than 10 mM EDTA, the enhancement of nisin by EDTA becomes reduced; the reduction of activation is significantly greater at pH 8.0 than at pH 5.0.

Example 7

Nisin and a Chelating Agent Against Gram Negative Bacteria (S. typhimurium)

The enhancement of the activity of nisin by EDTA towards Gram negative bacteria in the presence of biological tissue was demonstrated with S. typhimurium on chicken muscle, and is shown in Table 7.

TABLE 7

|          |     | Nisin  | EDTA(mM) % Survival[a] at 2 hours | | | | | | | | |
| Test #   | pH  | µg/ml  | 0    | 0.1 | 0.3    | 1        | 3      | 10    | 20   | 30   | 100  |
| ---      | --- | ---    | ---  | --- | ---    | ---      | ---    | ---   | ---  | ---  | ---  |
| 1        | 5.0 | 0      | 11.8 | -   | -      | -        | -      | 6.4   | -    | -    | -    |
| 2        | 5.0 | 300    | 0.1  | 0.2 | 0.05   | 0.01     | 0.003  | 0.016 | 0.03 | 0.02 | 0.07 |
| 3        | 8.0 | 0      | 100  | -   | -      | -        | -      | 5.2   | -    | -    | -    |
| 4        | 8.0 | 300    | 7.5  | 0.1 | 0.02   | 0.02     | 0.09   | 0.47  | 0.5  | -    | 2.2  |
| 5        | 8.0 | 300[b] | 0.02 | 0.09| 0.0002 | $<10^{-4}$ | 0.0004 | 0.003 | -    | 0.03 | 0.09 |

a Unadhered cells

b Contains 1% Bovine serum albumin (BSA)

Incubations were performed in either 50mM sodium acetate, pH 5.0, or 20 mM Tris, pH 8.0 at 37°C.

Cubes of chicken muscle were cleansed with sodium hypochlorite and povidone iodine prior to use. To inoculate the tissue, the cubes of chicken muscle were dipped into a $10^8$ cells/ml suspension of S. typhimurium in 20 mM Tris HCl, pH 8.0. Excess moisture was removed from dipped cubes by tapping. The chicken samples were placed into sufficient buffer containing the nisin composition to cover the tissue and incubated for 2 hours at 37°C after which the tissue was removed to sufficient Phage buffer to cover the

12

tissue. The bacteria remaining in the test solution were collected by centrifugation, washed with Phage buffer, and combined with bacteria washed from the tissue by Phage buffer. The combined samples (termed "unadhered" cells) were serially diluted and 100 $\mu$l aliquots were plated for determination of surviving bacteria.

In tests #1 and #3 (Table 7), in the absence of nisin at either pH 5 or pH 8, EDTA alone has no significant effect on the survival of S. typhimurium. In tests #2 and #4 (Table 7), however, where 300 $\mu$g/ml nisin was present, the bactericides exhibited substantial activity towards S. typhimurium on chicken muscle at both pH 5.0 and pH 8.0.

The enhancement of nisin by EDTA was concentration dependent, the optimal concentration being in the range 0.3mM to 10mM EDTA at both pH values 5.0 and 8.0. At concentrations greater than 10mM EDTA at pH 8.0, the activation of nisin by EDTA is reduced. However, as is shown in test #5 (Table 7), in the presence of 1.0 % bovine serum albumin at pH 8.0, the efficacy of nisin towards S. typhimurium on chicken muscle is expressed throughout the range of EDTA concentrations up to 100mM.

Thus, bactericides containing nisin and low concentrations of chelating agent, such as EDTA in the range 0.1mM to 20mM, can be extremely effective for the elimination or prevention of contamination of food by Gram negative bacteria.

Example 8

Titration of Nisin Activity Against Gram Negative Bacteria (S. typhimurium)

At the optimal concentration of chelating agent, the efficacy of the bactericide in Tris buffer towards Gram negative bacteria was demonstrated to be substantial, as is shown in Table 8.

TABLE 8

| Test # | Initial Viable Bacterial Count | EDTA (mM) | BSA % | % Survival at 2 hours | | | | | | | | |
|---|---|---|---|---|---|---|---|---|---|---|---|---|
| | | | | Nisin $\mu$g/ml | | | | | | | | |
| | | | | 0 | 0.1 | 0.3 | 1.0 | 3.0 | 10 | 30 | 100 | |
| 1 | $6 \times 10^6$ | 0 | 0 | 100 | - | - | - | - | 51.3 | - | 1.6 | |
| 2 | $6 \times 10^6$ | 1.0 | 1.0 | 63 | 0.7 | 0.08 | 0.01 | 0.05 | 0.01 | $< 10^{-4}$ | - | |

In test #2 (Table 8), it can be seen that as little as 0.3 $\mu$g/ml of nisin, with 1.0 mM EDTA in 20mM Tris at pH 8.0 in the presence of 1% bovine serum albumin (BSA), significantly reduced the survival of S. typhimurium. The bactericide is as active towards Gram negative bacteria as nisin alone is towards Gram positive Streptococci.

14

Example 9

Titration of Nisin Activity Against Gram Negative Bacteria (S. typhimurium)

At the optimal concentration of chelating agent, the efficacy of a bactericide towards Gram negative bacteria in the presence of biological tissue was demonstrated with S. typhimurium on chicken muscle, and is shown in Table 9.

TABLE 9

| Test # | pH | Initial Viable Bacterial Count | EDTA (mM) | BSA ( % ) | Nisin $\mu$ g/ml | | | | |
|--------|-----|------------------|-----------|-----------|-----|------|-------|-------|-------|
| | | | | | 0 | 10 | 100 | 200 | 300 |
| | | | | | % Survival at 2 hours | | | | |
| 1 | 8.0 | $3 \times 10^7$ | 0 | 0 | 100 | – | – | – | – |
| 2 | 8.0 | $3 \times 10^7$ | 1.0 | 1.0 | 27 | 0.26 | 0.008 | 0.007 | 0.006 |

Cubes of chicken muscle were cleansed with sodium hypochlorite and povidone iodine prior to use. To inoculate the tissue, the cubes of chicken muscle were dipped into a $10^8$ cells/ml suspension of S. typhimurium in 20 mM Tris HCl, pH 8.0. Excess moisture was removed from dipped cubes by tapping. The tissue was placed into sufficient buffer containing the nisin compositions to cover the tissue, and incubated for 2 hours at 37°C after which the tissue was removed to sufficient Phage buffer to cover the tissue. The bacteria remaining in the test solution were collected by centrifugation, washed with Phage buffer, and combined with bacteria washed from the tissue by Phage buffer. The combined samples (termed "unadhered" cells) were serially diluted and 100$\mu$l aliquots were plated for determination of surviving bacteria.

Example 10

Nisin EDTA and Methyl Paraben Activity Against Gram Negative Bacteria (S. typhimurium)

A bactericide containing nisin and EDTA, when combined with a known food preservative, methyl paraben, was demonstrated to be exceptionally effective towards Gram negative bacteria, as shown in Table 10.

TABLE 10

| Test # | Initial Viable Bacterial Count | Nisin g/ml | EDTA[b] (mM) | % Methyl Paraben | | |
|--------|-------------------------------|------------|--------------|-----|-----|-----|
| | | | | 0 | 0.1 | 1.0 |
| | | | | % Survival[c] at 2 hours | | |
| 1 | $3 \times 10^6$ | 0 | 10 | 11.8 | 1.0 | $10^{-4}$ |
| 2 | $3 \times 10^6$ | 300 | 10 | 0.03 | $<10^{-3}$ | $<10^{-4}$ |

[b] 50 mM Na acetate buffer, pH 5.0
[c] Unadhered cells

Cubes of chicken muscle were cleaned with sodium hypochlorite and povidone iodine prior to use. To inoculate the tissue, the cubes of chicken muscle were dipped into a $10^8$ cells/ml suspension of S. typhimurium in 50 mM sodium acetate buffer, pH 5.0. Excess moisture was removed from dipped cubes by tapping. The tissue was placed into sufficient buffer containing nisin compositions to cover the tissue, and incubated for 2 hours at 37°C after which the tissue was removed to sufficient Phage buffer to cover the tissue. The bacteria remaining in the test solution were collected by centrifugation, washed with Phage buffer, and combined with bacteria washed from the tissue by Phage buffer. The combined samples (termed "unadhered" cells) were serially diluted and 100μl aliquots were plated for determination of surviving bacteria.

In test #1 (Table 10), methyl paraben in the presence of 10 mM EDTA was shown to be effective towards S. typhimurium only at a concentration of 1.0%. In test #2 (Table 10), however, in the presence of 300 μg/ml nisin, the effectiveness of methyl paraben and nisin towards S. typhimurium was substantially improved.

The compositions containing nisin and EDTA significantly improve the utility of the food preservative methyl paraben. Furthermore, the bactericides may lead to substantial reductions in the concentrations, or eliminate the need for these commonly recognized, though less desirable, food preservatives such as methyl paraben.

Example 11

Nisin and Chelating Agent Activity Against Gram Positive Bacteria (Staphylococcus aureus)

The activation of nisin by a chelating agent is pH-dependent. The data in Table 11 confirm that at pH 5.0, nisin is somewhat more bactericidal towards S. aureus than is nisin at pH 8.0. At pH 5.0, EDTA does not enhance nisin activity towards S. aureus and at concentrations of EDTA greater than 10 mM, EDTA is inhibitory to the bactericidal activity of nisin. However, the bactericidal activity of nisin activated by EDTA at pH 8.0 is significantly greater than the bactericidal activity of nisin alone, or in combination with EDTA at pH 5.0.

## Table 11

### Influence of pH on the Effects of EDTA on Nisin Bactericidal Activity towards Staphyloccus aureus

| pH | Nisin $\mu$g/ml | EDTA mM | | | | | | | |
|----|-----------------|---------|-----|-----|-----|-----|----|----|-----|
| | | 0 | 0.1 | 0.3 | 1.0 | 3.0 | 10 | 30 | 100 |
| | | % Survival 2 hr[a] | | | | | | | |
| 8.0 | 0 | 100 | – | 100 | 81 | 100 | 100 | 100 | – |
| 8.0 | 3.0 | 7.4 | 0.03 | 0.01 | 0.2 | 0.4 | 3 | 56 | – |
| 5.0 | 0 | 100 | – | – | – | 100 | – | – | – |
| 5.0 | 3.0 | 0.6 | 1.0 | 1.3 | 1.4 | 1.8 | – | 34 | 80 |

[a] Initial viable count: $8.0 \times 10^6$ cfu/ml

Incubations were performed in 50 mM sodium acetate buffer, pH 5.0 or 20 mM Tris-HCl buffer, pH 8.0 at 37°C.

The bactericidal activity of nisin alone is reported (see Hurst) to be greatest at pH 5.0 or lower, and data presented in Table 11 support this. On the basis of this information it was believed that the bactericidal activation of nisin by EDTA towards S. aureus would likewise be greatest at lower pH. However, as can be seen in Table 11 and contrary to expectations (see Table 6), EDTA was not observed to enhance nisin

16

activity towards Gram positive bacteria at pH 5.0. However, inhibition of nisin activity by high concentrations of EDTA was still observed at pH 5.0. Thus, the activation of nisin by a chelating agent occurs only within a range of chelator concentrations and, with respect to Gram positive bacteria, is dependent upon pH with the preferred pH range greater than pH 5.0.

Example 12

Nisin and Chelating Agent Activity Against Gram Positive Bacteria

The effects of EDTA on the bactericidal activity of nisin at pH 8.0 are not limited to S. aureus, an important human pathogen, but are also observed with Streptococcus mutans, responsible for dental plaque (Table 12A), Listeria monocytogenes, a foodborne pathogen (Table 12B), and with a mixed population of axillary Coryneform bacteria, contributors to body odor (Table 12C).

## Table 12A

### The Effects of EDTA on the Bactericidal Activity of Nisin towards Streptococcus mutans

| pH | Nisin $\mu$g/ml | EDTA mM | | | | | | | | |
|----|-----|-----|------|-----|-----|-----|-----|-----|-----|-----|
| | | 0 | 0.01 | 0.1 | 0.3 | 1.0 | 3.0 | 10 | 30 | 100 |
| | | % Survival after 2 hr[a] | | | | | | | | |
| 8.0 | 0 | 100 | − | − | − | − | − | − | − | − |
| 8.0 | 0.1 | 4.3 | 1.8 | 0.04 | 0.02 | 0.06 | 1 | 25 | 100 | 100 |

[a] Initial viable count: $6.0 \times 10^6$ cfu/ml

Incubations were performed in 20 mM Tris-HCl, pH 8.0 at 37°C.

## Table 12B

### The Effects of EDTA on the Bactericidal
### Activity towards Listeria monocytogenes

| pH | Nisin $\mu$g/ml | EDTA mM | | | | | | | |
|----|----------|-----|-----|-----|-----|-----|-----|-----|-----|
| | | 0 | 0.1 | 0.3 | 1.0 | 3.0 | 10 | 30 | 100 |
| | | % Survival after 2hr[a] | | | | | | | |
| 8.0 | 0 | 100 | - | - | 84 | - | - | - | - |
| 8.0 | 3.0 | 0.71 | 0.04 | 0.04 | 0.02 | 0.1 | 0.64 | 10 | 14 |

[a] Initial viable count: $6.0 \times 10^6$ cfu/ml

Incubations were performed in 20 mM Tris-HCl, pH 8.0 at 37°C.

## Table 12C

| The Effects of EDTA on Nisin Bactericidal Activity towards Coryneform bacteria | | | | | | | |
|------|-----------|-----|-----|-----|-----|-----|-----|
| pH | Nisin g/ml | EDTA mM | | | | | |
| | | 0 | 0.1 | 0.3 | 1.0 | 3.0 | 10 |
| | | % Survival 2hr[a] | | | | | |
| 8.0 | 0 | 100 | - | 4.6 | 3.6 | 8 | 36 |
| 8.0 | 3 | 0.22 | 0.03 | 0.0009 | 0.1 | -- | 0.16 |

[a] Initial viable count: $1.0 \times 10^6$ cfu/ml
Incubations were performed in 20 mM Tris-HCl, pH 8.0 at 37°C.

### Example 13

Rapid Bactericidal Activity of Nisin Activated by Chelator

The bactericide comprising nisin with EDTA is rapidly bactericidal as is illustrated by the data presented in Table 13A. Suspensions of the Gram positive bacterium S. mutans at $10^7$ cells/ml were incubated in 20 mM Tris buffer, pH 7.3 at 37°C with a range of concentrations of nisin activated by 1 mM EDTA. The suspensions were incubated for various times ranging from 0.5 to 60 minutes with the bactericides. The bactericidal efficacy of the bactericides was estimated by determining the percent survival of bacteria. Enhanced by EDTA, as little as 10 $\mu$g/ml of the nisin in this formulation is able to reduce the bacterial load by 6 logs within 1 minute.

Rapid bactericidal activity is a prerequisite for effective disinfection. Thus, the compositions are predicted to be effective bactericides particularly as demonstrated here, as a component of a mouthwash, rinse, toothpaste, or other similar dentrifice active against plaque forming S. mutans.

The activity of nisin enhanced by EDTA against Gram negative bacteria after 2-3 hours was shown in Examples 1-7. Rapid bactericidal activity of nisin enhanced by EDTA is also seen towards Gram negative

18

bacteria and this is illustrated by the data in Table 13B.

Table 13A

| Kinetics of Bactericidal Activity towards Streptococcus mutans of Nisin Enhanced by EDTA | | | | | | |
|---|---|---|---|---|---|---|
| Incubation Time (Minutes) | Nisin $\mu$g/ml with 1.0 mM EDTA | | | | | |
| | 0 | 1 | 3 | 10 | 30 | 100 |
| | % Survival[a] | | | | | |
| 0.5 | - | - | - | - | - | $<10^{-4}$ |
| 1 | - | - | - | $<10^{-4}$ | $<10^{-4}$ | $<10^{-4}$ |
| 3 | 100 | 0.5 | 0.002 | $<10^{-4}$ | $<10^{-4}$ | - |
| 15 | - | 0.03 | $<10^{-4}$ | $<10^{-4}$ | - | - |
| 30 | - | - | $<10^{-4}$ | - | - | - |
| 60 | 100 | 0.003 | - | - | - | - |

[a] Control viable cell count: $1.0 \times 10^7$ cfu/ml
Incubations were performed in 20 mM Tris-HCl, pH 7.3 at 37°C.

TABLE 13B

| Rapid Bactericidal Activity towards Escherichia coli of Nisin Enhanced by EDTA | | | | | | | |
|---|---|---|---|---|---|---|---|
| mM EDTA | Nisin $\mu$g/ml | | | | | | |
| | 0 | 0.3 | 1.0 | 3 | 10 | 30 | 100 |
| | % survival at 1 min[a] | | | | | | |
| 1.0 | 100 | 100 | 56 | 0.37 | 0.013 | 0.015 | 0.008 |

[a] Initial viable count: $1.0 \times 10^7$ cfu/ml
Incubations were performed in 20 mM Tris, pH 7.0 at 37°C.

Example 14

Effect of Divalent Cations on EDTA Enhancement of Nisin Activity

Divalent cations bind to EDTA and other chelating agents and would be expected to neutralize the activation of nisin by EDTA. However, as can be seen by the data in Table 14, the bactericidal activity of nisin against S. mutans is enhanced by 1 mM EDTA even in the presence of 1 mM $Ca^{2+}$ ion; only above 3 mM was $Ca^{2+}$ ion inhibitory to EDTA-activated nisin. This is particularly important in mouthwash applications where calcium ion concentrations are relevant.

TABLE 14

| Rapid Bactericidal Activity towards Streptococcus mutans of Nisin Activated by EDTA in the presence of Divalent Cation | | | | | | |
|---|---|---|---|---|---|---|
| Nisin | CaCl$_2$ mM | | | | | |
|  | 0 | 0.1 | 0.3 | 1.0 | 3 | 10 |
|  | % survival at 1 min.[a] | | | | | |
| 0 | 100 |  |  |  |  |  |
| 3 | 2.9 |  |  |  |  |  |
| 3$^E$ | 0.0042 | 0.0042 | 0.052 |  |  | 18 |
| 30$^E$ | 0.0019 |  | 0.0003 | 0.0004 | 0.06 | 6.8 |
| 100$^E$ | <10$^{-4}$ |  | <10$^{-4}$ | <10$^{-4}$ | 0.0001 | 1.5 |

$^E$ 1 mM Na$_2$EDTA

[a] Initial viable count $1.0 \times 10^2$ cfu/ml.

Incubations performed in 10% Fetal Calf Serum at 37°C.

**Claims**

1. A composition for enhancing the performance of a lanthionine-containing bacteriocin, comprising a lanthionine-containing bacteriocin other than nisin and subtilin, characterized in that it further comprises a chelating agent.

2. A composition for enhancing the performance of a lanthionine-containing bacteriocin which is nisin or subtilin, comprising nisin or subtilin, characterized in that it further comprises a chelating agent.

3. A composition according to Claim 1, characterized in that the lanthionine-containing bacteriocin is epidermin, cinnamycin, duramycin, ancovenin or Pep 5.

4. A composition according to Claim 1, characterized in that the concentration of lanthionine-containing bacteriocin is from 0.1 to 300 micrograms/ml. and the concentration of chelating agent is from 0.1 to 20 mM.

5. A composition according to Claim 2, characterized in that it contains from 0.1 to 300 micrograms/ml. of nisin and in that the concentration of chelating agent is from 0.1 to 20 mM.

6. A composition according to Claim 1, 3 or 4, characterized in that the chelating agent is EDTA.

7. A composition according to Claim 2 or 5, characterized in that the chelating agent is EDTA.

8. A composition according to Claim 1, 3, 4 or 6, characterized in that it further comprises a surfactant.

9. A composition according to Claim 2, 5 or 7, characterized in that it further comprises a surfactant.

10. A composition according to Claim 8 or 9, characterized in that the surfactant is selected from non-ionic surfactants, fatty acids, quaternary compounds and amphoteric surfactants.

11. A composition according to Claim 9, characterized in that the surfactant is non-ionic.

12. A composition according to Claim 10, characterized in that the surfactant is a glyceride.

13. A composition according to Claim 11, characterized in that the surfactant is a glyceride.

14. A composition according to Claim 1, 3, 4, 6, 8, 10 or 12 in the form of a solution.

**15.** A composition according to Claim 2, 5, 7, 9, or 11, in the form of a solution.

**16.** A composition according to Claim 14, characterized in that the solvent is an aqueous buffer imparting a pH of from 5 to 8.

**17.** A composition according to Claim 15, characterized in that the solvent is an aqueous buffer imparting a pH of from 5 to 8.

**18.** A composition for enhancing the performance of a lanthionine-containing bacteriocin and for medicinal use as a bactericide, comprising a lanthionine-containing bacteriocin and a chelating agent.

**19.** A composition according to Claim 18, characterized in that it further comprises a surfactant.

**20.** A composition according to Claim 18 or 19, for use in the oral cavity.

**21.** A composition according to Claim 20, for use as an oral rinse.

**22.** A composition according to Claim 18 or 19, for use as a component of toothpaste.

**23.** A composition according to Claim 18 or 19, for use in a wound dressing or as a skin disinfectant.

**24.** A composition according to Claim 18 , 19 or 20, characterized in that the lanthionine-containing bacteriocin is nisin or subtilin.

**25.** A composition according to Claim 21 or 22, characterized in that the lanthionine-containing bacteriocin is nisin or subtilin.

**26.** A composition according to Claim 18 or 19, for use as a skin disinfectant, characterized in that the lanthionine-containing bacteriocin is nisin or subtilin.

**27.** A composition according to Claim 24 or 26, characterized in that the chelating agent is an alkylenediamine tetraacetate, EGTA or citrate.

**28.** A composition according to any one of Claims 18 to 27, characterized in that the chelating agent is EDTA, monocalcium EDTA or monocalcium disodium EDTA.

**29.** A composition according to Claim 27, characterized in that the chelating agent is EDTA.

**30.** A method of treating food characterized by the use in combination of a lanthionine-containing bacteriocin other than nisin and subtilin and a chelating agent to enhance the performance of the lanthionine-containing bacteriocin.

**31.** A method of treating food characterized by the use in combination of a lanthionine-containing bacteriocin which is nisin or subtilin and a chelating agent to enhance the performance of the nisin or subtilin.

**32.** A method according to Claim 30, characterized in that the chelating agent is EDTA.

**33.** A method according to Claim 31, characterized in that the chelating agent is EDTA.

**34.** A method according to Claim 30 or 32, characterized in that the combination further comprises a surfactant.

**35.** A method according to Claim 31 or 33, characterized in that the combination further comprises a surfactant.

**36.** Use in combination of (1) a lanthionine-containing bacteriocin other than nisin or subtilin and (2) a chelating agent, as a bactericide effective against Gram-negative bacteria and a broad range of Gram-

21

positive bacteria, whereby the performance of the lanthionine-containing bacteriocin is enhanced, other than for medicinal purposes.

37. Use in combination of (1) a lanthionine-containing bacteriocin which is nisin or subtilin and (2) a chelating agent, as a bactericide effective against Gram-negative bacteria and a broad range of Gram-positive bacteria, whereby the performance of the nisin or subtilin is enhanced, other than for medicinal purposes.

38. Use according to Claim 36 for the treatment of food.

39. Use according to Claim 37 for the treatment of food.

40. Use according to Claim 36, for cleaning poultry, fish, meats and vegetables, and dairy and food processing equipment.

41. Use according to Claim 36, for cleaning poultry, fish, meats and vegetables, and dairy and food processing equipment.

42. Use according to Claim 36, 38 or 40 against Gram-negative bacteria.

43. Use according to Claim 37, 39 or 41 against Gram-negative bacteria.

44. Use according to Claim 42 or 43, characterized in that the bacteria comprise Salmonella typhimurium, Escherichia coli, Pseudomonas aeruginosa, Bacteroides gingivalis, Actinobacillus actinomycetescomitans or Klebsiella pneumoniae.

45. Use according to Claim 43, characterized in that the bacteria comprise Salmonella typhimurium, Escherichia coli or Klebsiella pneumoniae.

46. Use according to any one of Claims 36 to 45, characterized in that the chelating agent is an alkylenediamine tetraacetate, EGTA or citrate.

47. Use according to Claim 37, 39, 41, 43 or 45, characterized in that the chelating agent is EDTA.

48. Use according to Claim 46, characterized in that the chelating agent is EDTA.

49. Use according to Claim 36, 38, 40, 42, 44, 46 or 48, characterized in that the combination further comprises a surfactant.

50. Use according to Claim 37, 39, 41, 43, 45 or 47, characterized in that the combination further comprises a surfactant.

**Patentansprüche**

1. Zusammensetzung zur Verstärkung der Leistung eines Lanthionin enthaltenden Bakteriocins mit einem von Nisin und Subtilin verschiedenen Lanthionin enthaltenden Bakteriocin, dadurch gekennzeichnet, daß sie ferner einen Chelatbildner enthält.

2. Zusammensetzung zur Verstärkung der Leistung eines Lanthionin enthaltenden Bakteriocins, welches Nisin oder Subtilin ist, enthaltend Nisin oder Subtilin, dadurch gekennzeichnet, daß sie ferner einen Chelatbildner enthält.

3. Zusammensetzung nach Anspruch 1, dadurch gekennzeichnet, daß das Lanthionin enthaltende Bakteriocin, Epidermin, Cinnamycin, Duramycin, Anconvenin oder Pep 5 ist.

4. Zusammensetzung nach Anspruch 1, dadurch gekennzeichnet, daß die Konzentration des Lanthionin enthaltenden Bakteriocins, 0,1 bis 300 mg/ml und Konzentration des Chelatbildners 0,1 bis 20 mM beträgt.

**5.** Zusammensetzung nach Anspruch 2, dadurch gekennzeichnet, daß sie 0,1 bis 300 mg/ml Nisin enthält und die Konzentration des Chelatbildners 0,1 bis 20 mM beträgt.

**6.** Zusammensetzung nach Anspruch 1, 3 oder 4, dadurch gekennzeichnet, daß der Chelatbildner EDTA ist.

**7.** Zusammensetzung nach Anspruch 2 oder 5, dadurch gekennzeichnet, daß der Chelatbildner EDTA ist.

**8.** Zusammensetzung nach Anspruch 1, 3 ,4 oder 6, dadurch gekennzeichnet, daß sie ferner ein Tensid enthält.

**9.** Zusammensetzung nach Anspruch 2, 5 oder 7, dadurch gekennzeichnet, daß sie ferner ein Tensid enthält.

**10.** Zusammensetzung nach Anspruch 8 oder 9, dadurch gekennzeichnet, daß das Tensid ausgewählt ist aus nichtionischen Tensiden, Fettsäuren, quartären Verbindungen und amphotären Tensiden.

**11.** Zusammensetzung nach Anspruch 9, dadurch gekennzeichnet, daß das Tensid ein nichtionisches Tensid ist.

**12.** Zusammensetzung nach Anspruch 10, dadurch gekennzeichnet, daß das Tensid ein Glycerid ist.

**13.** Zusammensetzung nach Anspruch 11, dadurch gekennzeichnet, daß das Tensid ein Glycerid ist.

**14.** Zusammensetzung nach Anspruch 1, 3, 4 , 6, 8, 10 oder 12 in Form einer Lösung.

**15.** Zusammensetzung nach Anspruch 2, 5, 7, 9 oder 11 in Form einer Lösung.

**16.** Zusammensetzung nach Anspruch 14 dadurch gekennzeichnet, daß das Lösemittel einen pH-Wert von 5 bis 8 verleihender, wäßriger Puffer ist.

**17.** Zusammensetzung nach Anspruch 15, dadurch gekennzeichnet, daß das Lösemittel einen pH-Wert von 5 bis 8 verleihender, wäßriger Puffer ist.

**18.** Zusammensetzung zur Verstärkung der Leistung eines Lanthionin enthaltenden Bakteriocins und zur medizinischen Verwendung als Baktericid, enthaltend ein Lanthionin enthaltendes Bakteriocin und einen Chelatbildner.

**19.** Zusammensetzung nach Anspruch 18, dadurch gekennzeichnet, daß sie ferner ein Tensid enthält.

**20.** Zusammensetzung nach Anspruch 18 oder 19 zur Verwendung in der Mundhöhle.

**21.** Zusammensetzung nach Anspruch 20 zur Verwendung als Mundspülmittel.

**22.** Zusammensetzung nach Anspruch 18 oder 19 zur Verwendung als Bestandteil einer Zahnpaste.

**23.** Zusammensetzung nach Anspruch 18 oder 19 zur Verwendung in einer Wundabdeckung oder als Hautdesinfektionsmittel.

**24.** Zusammensetzung nach Anspruch 18, 19 oder 20, dadurch gekennzeichnet, daß das Lanthionin enthaltende Bakteriocin, Nisin oder Subtilin ist.

**25.** Zusammensetzung nach Anspruch 21 oder 22, dadurch gekennzeichnet, daß das Lanthionin enthaltende Bakteriocin, Nisin oder Subtilin ist.

**26.** Zusammensetzung nach Anspruch 18 oder 19 zur Verwendung als Hautdesinfektionsmittel, dadurch gekennzeichnet, daß das Lanthionin enthaltende Bakteriocin, Nisin oder Subtilin ist.

27. Zusammensetzung nach Anspruch 24 oder 26, dadurch gekennzeichnet, daß der Chelatbildner ein Alkylindiamintetraacetat oder Citrat ist.

28. Zusammensetzung nach einem der Ansprüche 18 bis 27, dadurch gekennzeichnet, daß der Chelatbildner EDTA, Monocalcium-EDTA oder Monocalcium-Dinatrium-EDTA ist.

29. Zusammensetzung nach Anspruch 27, dadurch gekennzeichnet, daß der Chelatbildner EDTA ist.

30. Verfahren zur Behandlung von Lebensmitteln durch die gemeinsame Verwendung mit einem von Nisin und Subtilin verschiedenen Lanthionin enthaltenden Bakteriocin und einem Chelatbildner zur Verstärkung der Leistung des Lanthionin enthaltenden Bakteriocins.

31. Verfahren zur Behandlung von Lebensmitteln durch die gemeinsame Verwendung eines Lanthionin enthaltenden Bakteriocins, welches Nisin oder Subtilin ist, und einem Chelatbildner zur Verstärkung der Leistung von Nisin oder Subtilin.

32. Verfahren nach Anspruch 30, dadurch gekennzeichnet, daß der Chelatbildner EDTA ist.

33. Verfahren nach Anspruch 31, dadurch gekennzeichnet, daß der Chelatbildner EDTA ist.

34. Verfahren nach Anspruch 30 oder 32, dadurch gekennzeichnet, daß die Zusammensetzung ferner ein Tensid enthält.

35. Verfahren nach Anspruch 31 oder 33, dadurch gekennzeichnet, daß die Zusammensetzung ferner ein Tensid enthält.

36. Verwendung in Kombination von (1) eines Lanthionin enthaltenden von Nisin oder Subtilin verschiedenen Bakteriocins und (2) eines Chelatbildners als wirksames Bakteriocid gegen gramnegative Bakterien und einen weiten Bereich von grampositiven Bakterien, wobei die Leistung des Lanthionin enthaltenden Bakteriocins verstärkt ist, für andere als medizinische Zwecke.

37. Verwendung in Kombination von (1) eines Lanthionin enthaltenden Bakteriocins, welches Nisin oder Subtilin ist, und (2) eines Chelatbildners als gegen gramnegative Bakterien und einen weiten Bereich von grampositiven Bakterien wirksames Bakteriocid, wobei die Leistung von Nisin oder Subtilin verstärkt ist, für andere als medizinische Zwecke.

38. Verwendung nach Anspruch 36 zur Behandlung von Lebensmitteln.

39. Verwendung nach Anspruch 37 zur Behandlung von Lebensmitteln.

40. Verwendung nach Anspruch 36 zur Reinigung von Geflügel, Fisch, Fleisch und Gemüse und Molkerei- und Lebensmittelausrüstungsgegenständen.

41. Verwendung nach Anspruch 36 zur Reinigung von Geflügel, Fisch, Fleisch und Gemüse und Molkerei- und Lebensmittelausrüstungsgegenständen.

42. Verwendung nach Anspruch 36, 38, oder 40 gegen gramnegative Bakterien.

43. Verwendung nach Anspruch 37, 39 oder 41 gegen gramnegative Bakterien.

44. Verwendung nach Anspruch 42 oder 43, dadurch gekennzeichnet, daß die Bakterien Salmonella typhimurium, Escherichia coli, Pseudomonas aeruginosa, Bactroides gingivalis, Actinobacillus actinomycetescomitans oder Klebsiella pneumoniae umfassen.

45. Verwendung nach Anspruch 43, dadurch gekennzeichnet, daß die Bakterien Salmonella typhimurium, Escherichia coli oder Klebsiella pneumoniae umfassen.

**46.** Verwendung nach einem der Ansprüche 36 bis 45, dadurch gekennzeichnet, daß der Chelatbildner ein Alkylendiamintetraacetat, EGTA oder Citrat ist.

**47.** Verwendung nach Anspruch 37, 39, 41, 43 oder 45, dadurch gekennzeichnet, daß der Chelatbildner EDTA ist.

**48.** Verwendung nach Anspruch 46, dadurch gekennzeichnet, daß der Chelatbildner EDTA ist.

**49.** Verwendung nach Anspruch 36, 38, 40, 42, 44, 46 oder 48, dadurch gekennzeichnet, daß die Kombination ferner ein Tensid enthält.

**50.** Verwendung nach Anspruch 37, 39, 41, 43, 45 oder 47, dadurch gekennzeichnet, daß die Kombination ferner ein Tensid enthält.

**Revendications**

**1.** Composition pour améliorer l'efficacité d'une bactériocine contenant de la lanthionine, comprenant une bactériocine contenant de la lanthionine autre que la nisine et la subtiline, caractérisée en ce qu'elle comprend de plus un agent chélateur.

**2.** Composition pour améliorer l'efficacité d'une bactériocine contenant de la lanthionine, laquelle est de la nisine et de la subtiline, comprenant de la nisine ou de la subtiline, caractérisée en ce qu'elle comprend de plus un agent chélateur.

**3.** Composition selon la revendication 1, caractérisée en ce que la bactériocine contenant de la lanthionine est de l'épidermine, de la cinnamycine, de la duramycine, de ancovenine ou Pep 5.

**4.** Composition selon la revendication 1, caractérisée en ce que la concentration de bactériocine contenant de la lanthionine est de 0,1 à 300 microgrammes/ml et que la concentration de l'agent chélateur est de 0,1 à 20 mM.

**5.** Composition selon la revendication 2, caractérisée en ce qu'elle contient de 0,1 à 300 microgrammes/ml de nisine et en ce que la concentration de l'agent chélateur est de 0,1 à 20 mM.

**6.** Composition selon la revendication 1, 3 ou 4, caractérisée en ce que l'agent chélateur est l'EDTA.

**7.** Composition selon la revendication 2 ou 5, caractérisée en ce que l'agent chélateur est l'EDTA.

**8.** Composition selon la revendication 1, 3, 4 ou 6, caractérisée en ce qu'elle comprend de plus un agent tensioactif.

**9.** Composition selon la revendication 2, 5 ou 7, caractérisée en ce qu'elle comprend de plus un agent tensioactif.

**10.** Composition selon la revendication 8 ou 9, caractérisée en ce que l'agent tensioactif est sélectionné parmi les agents tensioactifs non ioniques, les acides gras, les composés quaternaires et les agents tensioactifs amphotériques.

**11.** Composition selon la revendication 9, caractérisée en ce que l'agent tensioactif est non ionique.

**12.** Composition selon la revendication 10, caractérisée en ce que l'agent tensioactif est un glycéride.

**13.** Composition selon la revendication 11, caractérisée en ce que l'agent tensioactif est un glycéride.

**14.** Composition selon la revendication 1, 3, 4, 6, 8, 10 ou 12 sous la forme d'une solution.

**15.** Composition selon la revendication 2, 5, 7, 9 ou 11 sous la forme d'une solution.

**16.** Composition selon la revendication 14, caractérisée en ce que le solvant est un tampon aqueux conférant un pH de 5 à 8.

**17.** Composition selon la revendication 15, caractérisée en ce que le solvant est un tampon aqueux conférant un pH de 5 à 8.

**18.** Composition pour améliorer l'efficacité d'une bactériocine contenant de la lanthionine et pour utilisation médicale comme bactéricide, comprenant une bactériocine contenant de la lanthionine et un agent chélateur.

**19.** Composition selon la revendication 18, caractérisée en ce qu'elle comprend de plus un agent tensioactif.

**20.** Composition selon la revendication 18 ou 19, pour utilisation dans la bouche.

**21.** Composition selon la revendication 20, pour utilisation pour le bain de bouche.

**22.** Composition selon la revendication 18 ou 19, pour utilisation comme composant d'une pâte à dentifrice.

**23.** Composition selon la revendication 18 ou 19, pour utilisation dans la désinfection de plaie ou comme désinfectant de peau.

**24.** Composition selon la revendication 18, 19 ou 20, caractérisée en ce que la bactériocine contenant de la lanthionine est de la nisine ou de la subtiline.

**25.** Composition selon la revendication 21 ou 22, caractérisée en ce que la bactériocine contenant de la lanthionine est de la nisine ou de la subtiline.

**26.** Composition selon la revendication 18 ou 19, pour utilisation comme un désinfectant de la peau, caractérisée en ce que la bactériocine contenant de la lanthionine est de la nisine ou de la subtiline.

**27.** Composition selon la revendication 24 ou 26, caractérisée en ce que l'agent chélateur est un tétraacétate alkylènediomine, un EGTA ou du citrate.

**28.** Composition selon l'une quelconque des revendications 18 à 27, caractérisée en ce que l'agent chélateur est l'EDTA, l'EDTA monocalcium ou l'EDTA monocalcium disodium.

**29.** Composition selon la revendication 27, caractérisée en ce que l'agent chélateur est l'EDTA.

**30.** Procédé pour traiter des aliments caractérisé par l'utilisation en combinaison d'une bactériocine contenant de la lanthionine autre que la nisine et la subtiline et un agent chélateur pour améliorer l'efficacité de la bactériocine contenant de la lanthionine.

**31.** Procédé pour traiter des aliments caractérisé par l'utilisation en combinaison d'une bactériocine contenant de la lanthionine qui est la nisine ou la subtiline et d'un agent chélateur pour améliorer l'activité de la nisine ou de la subtiline.

**32.** Procédé selon la revendication 30, caractérisé en ce que l'agent chélateur est l'EDTA.

**33.** Procédé selon la revendication 31, caractérisé en ce que l'agent chélateur est l'EDTA.

**34.** Procédé selon la revendication 30 ou 32, caractérisé en ce que la combinaison comprend de plus un agent tensioactif.

**35.** Procédé selon la revendication 31 ou 33, caractérisé en ce que la combinaison comprend de plus un agent tensioactif.

36. Utilisation en combinaison (1) d'une bactériocine contenant de la lanthionine autre que la nisine ou la subtiline et (2) d'un agent chélateur, comme bactéricine efficace contre les bactéries à Gram négatif et une plage importante de bactéries à Gram positif, d'où il résulte que l'activité de la bactériocine contenant de la lanthionine est améliorée, pour utilisation autre que médicale.

37. Utilisation en combinaison (1) d'une bactériocine contenant de la lanthionine qui est de la nisine ou de la subtiline et (2) d'un agent chélateur, en tant que bactéricine efficace contre les bactéries à Gram négatif et une plage importante de bactéries à Gram positif, d'où il résulte que l'activité de la nisine ou de la subtiline est améliorée, pour utilisation autre médicale.

38. Utilisation selon la revendication 36 pour le traitement des aliments.

39. Utilisation selon la revendication 37 pour le traitement des aliments.

40. Utilisation selon la revendication 36, pour nettoyer la volaille, poissons, viandes et légumes et les équipements de transformation de produits laitiers et d'aliments.

41. Utilisation selon la revendication 36, pour nettoyer la volaille, poissons, viandes et légumes et équipements de traitement de produits laitiers et aliments.

42. Utilisation selon la revendication 36, 38 ou 40, contre les bactéries à Gram négatif.

43. Utilisation selon la revendication 37, 39 ou 41 contre les bactéries à Gram négatif.

44. Utilisation selon la revendication 42 ou 43, caractérisée en ce que les bactéries comprennent Salmonella typhimurium, Escherichia coli, Pseudomonas aeruginosa, Bacteroides gingivalis, Actinobacillus actinomycetescomitans ou Klebsiella pneumoniae.

45. Utilisation selon la revendication 43, caractérisée en ce que les bactéries comprennent Salmonella typhimurium, Escherichia coli ou Klebsiella pneumoniae.

46. Utilisation selon l'une quelconque des revendications 36 à 45, caractérisée en ce que l'agent chélateur est un tétraacétate alkylènediamine, d'EGTA ou du citrate.

47. Utilisation selon la revendication 37, 39, 41, 43 ou 45, caractérisée en ce que l'agent chélateur est l'EDTA.

48. Utilisation selon la revendication 46, caractérisée en ce que l'agent chélateur est l'EDTA.

49. Utilisation selon la revendication 36, 38, 40, 42, 44, 46 ou 48, caractérisée en ce que la combinaison comprend de plus un agent tensioactif.

50. Utilisation selon la revendication 37, 39, 41, 43, 45 ou 47, caractérisée en ce que la combinaison comprend de plus un agent tensioactif.